# FASCICULE DE BREVET EUROPEEN

(11) **EP 1 674 072 B1**
(45) Date de publication et mention de la délivrance du brevet: **04.06.2014**
(21) Numéro de dépôt: 05292332.3
(22) Date de dépôt: 03.11.2005
(51) Int. Cl.: A61K 8/42, A61Q 19/08

(54) **Utilisation de composés d'urée pour lutter contre les signes de vieillissement cutané**
Verwendung von Harnstoffverbindungen im Kampf gegen Anzeichen der Hautalterung
Use of urea compounds to combat indications of skin ageing

(30) Priorité: 04.11.2004 FR 0411783
(43) Date de publication de la demande: 28.06.2006
(73) Titulaire: L'Oréal, 75008 Paris (FR)
(72) Inventeur: Bernard, Dominique, 75015 Paris (FR); Simonetti, Lucie, Vincennes 94300 (FR)
(74) Mandataire: Brohmi, Karim

(56) Documents cités:
- EP-A2- 0 585 130
- EP-A2- 1 281 396
- DE-A1- 2 703 185
- FR-A1- 2 848 116
- ANONYMOUS: "Enhancing the deposition of benefit agents from a surfactant system onto hair and skin" IP.COM JOURNAL, IP.COM INC., WEST HENRIETTA, NY, US, 14 décembre 2004 (2004-12-14), XP013022540 ISSN: 1533-0001

## Description

La présente invention se rapporte à l'utilisation d'au moins un composé d'urée hydroxylé pour lutter contre les signes particuliers du vieillissement de la peau et des muqueuses.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules, un amincissement de l'épiderme et/ou un aspect de peau molle et flétrie. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

La peau est constituée de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures appelé "fonction barrière". L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin la couche cornée (ou *stratum corneum),* constituée d'un ensemble de couches de kératinocytes au stade terminal de leur différenciation appelés cornéocytes. Les cornéocytes sont des cellules anucléées principalement constituées d'une matière fibreuse contenant des cytokératines, entourée d'une enveloppe cornée.
Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.
La cohésion entre l'épiderme et le derme est assurée par la jonction dermo-épidermique.

Il y a en permanence dans l'épiderme une production de nouveaux kératinocytes pour compenser la perte en continu de cellules épidermiques au niveau de la couche cornée.

Cependant, au cours du vieillissement, la prolifération et la différenciation épidermique peuvent être perturbées de façon physiologique, et on peut observer une tendance au déséquilibre entre ces deux mécanismes.

En outre, il a été démontré un déclin du système protéasome avec l'age (Friguet et al., 2002, Scientific World Journal). Ceci peut être en partie relié à une accumulation de protéines oxydées donc à un dysfonctionnement cellulaire (Dunlop, Rodgers et al. 2002; Szweda, Friguet et al. 2002). Pour combler ce déficit une stimulation des activités serait donc souhaitable.

Il a été avancé que le pool d'acides aminés libres du stratum comeum diminuait avec l'état de sécheresse cutanée (Tanaka, Okada et al. 1998) qui est, nous amplifié chez les peaux âgées. Ces acides aminés ont pour origine la protéolyse de la filaggrine. On peut donc naturellement compenser ce déficit de protéolyse de la filaggrine par une stimulation des activités protéasiques de dégradation de cette protéine.

Enfin, la maturation de l'EC (enveloppe cornée) qui peut être altérée avec l'âge est sous le contrôle d'activités transglutaminases elles-mêmes activées par une protéolyse d'un précurseur; l'activation de protéases impliquées dans ce processing serait donc utile.

Il est donc toujours souhaitable de disposer de nouveaux moyens de lutter contre l'un ou plusieurs de ces phénomènes afin de prévenir, retarder ou diminuer les signes liés au vieillissement de la peau ou des muqueuses.

De manière inattendue, il a été trouvé que des dérivés de l'urée présentent une activité de stimulation des protéases de la peau.

C'est pourquoi la présente invention a pour objet l'utilisation d'au moins un composé de formule (I) suivante 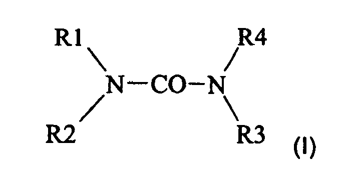 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères, dans une composition contenant un milieu physiologiquement acceptable, comme agent pour prévenir et/ou diminuer les signes du vieillissement de la peau et/ou des muqueuses.

Les composés de formule (I), leurs sels, leurs solvats et/ou leurs isomères seront notamment utiles comme agent pour réparer ou lutter contre le vieillissement de la peau, qu'il soit photo induit ou chronologique ou pour réduire les pigmentations actiniques.

Pour les composés de formule (I) :
- De préférence R1 désigne un groupe hydroxyalkyle en C2-C6 et R2, R3, R4 désignent, indépendamment l'un de l'autre, un atome d'hydrogène ou un groupe alkyle en C1-C4 ;
- Préférentiellement R1 désigne un groupe hydroxyalkyle en C2-C6 comprenant de 1 à 5 groupes hydroxyles, notamment 1 groupe hydroxyle, et R2, R3, R4 désignent un atome d'hydrogène ;
- Plus préférentiellement, R1 désigne un groupe hydroxyalkyle en C2-C4 comprenant 1 groupe hydroxyle et R2, R3, R4 désignent un atome d'hydrogène.

Parmi les groupes alkyle, on peut citer les groupes méthyle, éthyle, n-propyle, isopropyle, n-butyle, iso-butyle et tert-butyle.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques.

Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée; le N-méthyl-N-(1,3,4,5,6-pentahydroxy-2-hexyl)- urée ; le N-méthyl-N'-(1-hydroxy-2-méthyl-2-propyl)- urée ; le N-(1-hydroxy-2-méthyl-2-propyl)- urée; le N-(1,3-dihydroxy-2-propyl)- urée ; le N-(tris-hydroxyméthyl-méthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée; le N,N-Bis-(2-hydroxyéthyl)-N'-propyl- urée ; le N,N-Bis-(2-hydroxypropyl)-N'-(2-hydroxyéthyl)- urée; le N-tert.Butyl-N'-(2-(hydroxyéthyl)-N'-(2-(hydroxypropyl)- urée; le N-(1,3-dihydroxy-2-propyl)-N'-(2-hydroxyéthyl)- urée; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)- urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société NATIONAL STARCH sous la dénomination commerciale Hydrovance® . Toutefois, à la connaissance de la demanderesse, ces composés n'avaient jamais été proposée pour stimuler les activités protéolytiques et lutter contre les signes du vieillissement.
Le composé de formule (I) peut notamment être présent dans la composition selon l'invention en une teneur allant de 0,1 % à 50 % en poids, par rapport au poids total de la composition, de préférence allant de 0,1 % à 20 % en poids, et préférentiellement allant de 0,1 % à 10 % en poids.

Un milieu physiologiquement acceptable est selon l'invention un milieu cosmétiquement ou pharmaceutiquement acceptable compatible avec la peau, les muqueuses, les ongles et/ou les cheveux.
Les compositions selon l'invention peuvent être appliquées sur les ongles, les cheveux et plus particulièrement sur la peau et les muqueuses. Il s'agit de préférence d'un milieu cosmétiquement acceptable, c'est-à-dire qui présente une couleur, une odeur et un toucher agréable et qui ne génèrent pas d'inconfort inacceptable.
Les compositions sont de préférence des compositions ou produits cosmétique. Par "produit cosmétique", on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).

Les compositions contenant un dérivé de formule (I) selon l'invention sont particulièrement avantageuses pour agir sur un ou plusieurs mécanismes épidermiques tels que la dégradation de protéine(s), l'activation d'enzyme(s), et/ou la régulation du phénomène de différenciation/prolifération épidermique.

Le composé de formule (I) ou les compositions le contenant seront particulièrement utiles pour stimuler l'activité des enzymes protéolytiques du stratum comeum, et donc combattre les désordres associés à une diminution de l'activité de ces enzymes liée à l'âge. Ces enzymes sont notamment choisies dans le groupe comprenant :
- Des protéases à sérine telles que la SCCE, la SCTE, la matriptase MT-SP1, les « proprotein convertases » ou la PEP1 (« profilaggrin endoproteinase 1 »);
- Des protéases à acide aspartique telles que les cathepsine D, E ou la SASPase (Locuslink 151516) ;
- Des protéases à cystéine telle que les cathepsine B, H, L , L2 , les calpaïnes ou la caspase 14 ;
- Des métalloprotéases telle que la MMP19 ou les carboxypeptidases.

Les protéases ont été longtemps caractérisées comme des enzymes de dégradation non spécifiques, associées au catabolisme des protéines. Cependant, il devient de plus en plus clair que la protéolyse peut représenter un mécanisme fin de contrôle de certains processus biologiques commandant par exemple une localisation particulière, l'activation ou l'inactivation d'autres enzymes, de cytokines, d'hormones, de facteurs de croissance, la conversion d'agonistes en antagonistes... Les protéases modulent donc directement des processus biologiques essentiels, tels que la réplication de l'ADN, la progression du cycle cellulaire, la prolifération, la différentiation et la migration cellulaires, la morphogenèse, l'apoptose. Les protéases et leur régulation sont à des points clefs de régulation de la physiologie épidermique. Différentes protéases appartenant à presque toutes les classes de protéases connues sont associées à la différenciation épidermique et en sont des éléments de régulation importants.
Selon l'invention, on peut ainsi lutter contre le déficit du système protéasome en stimulant les activités de ses protéases. On peut améliorer la dégradation des protéines oxydées et/ou améliorer la maturation de l'enveloppe cornée dans une peau et/ou des muqueuses âgées.
En particulier, les composés de formule (I) ou les compositions les contenant seront utiles pour stimuler l'activité d'au moins une protéase acide du stratum corneum. Il s'agira en particulier des protéases à acide aspartique telles que celles décrites dans la demande WO 04/007548.
Les composés de formule (I) sont également utiles selon l'invention pour activer les enzymes d'activation des transglutaminases, et donc favoriser le clivage du précurseur inactif en forme active des transglutaminases.

Selon l'invention, les dérivés d'urée de formule (I) sont ainsi utiles comme agent pour réguler la différenciation épidermique, en particulier dans une peau âgée, et/ou pour favoriser la dégradation de la filaggrine et inhiber la diminution de la quantité d'acides aminés libres dans le stratum comeum liée à l'âge. L'invention comprend également l'utilisation de tels composés en vue de diminuer l'accumulation de protéines anormales liée au vieillissement.
D'une manière générale, toute composition de l'invention peut être ingérée, injectée ou appliquée sur la peau (sur toute zone cutanée du corps) ou sur les muqueuses (buccale, jugale, gingivale, génitale, conjonctivale, ...).
De préférence, une composition de l'invention est appliquée sur la peau ou les muqueuses.
Selon le mode d'administration considéré, elle peut se présenter sous toutes les formes galéniques normalement utilisées.

Pour une application topique sur la peau, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles.
Pour l'injection, la composition peut se présenter sous forme de lotions aqueuses, huileuses ou sous forme de sérums. Pour les yeux, elle peut se présenter sous forme de gouttes et pour l'ingestion, elle peut se présenter sous forme de capsules, de granulés, de sirops ou de comprimés.
Les quantités des différents constituants des compositions selon l'invention sont celles classiquement utilisées dans les domaines considérés.
Dans le domaine de la cosmétique, ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens et les prurits sévères.
Les compositions selon l'invention peuvent également consister en des préparations solides constituant des savons ou des pains de nettoyage.
Les compositions peuvent aussi être conditionnées sous forme de composition pour aérosol comprenant également un agent propulseur sous pression.
Une composition selon l'invention peut aussi être une composition pour les soins du cuir chevelu, et notamment un shampoing, une lotion de mise en plis, une lotion traitante, une crème ou un gel coiffant, une composition de teintures (notamment teintures d'oxydation) éventuellement sous forme de shampoings colorants, de lotions restructurantes pour les cheveux, une composition de permanente (notamment une composition pour le premier temps d'une permanente), une lotion ou un gel antichute, un shampoing antiparasitaire, antipelliculaire.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.
Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables dans l'invention, on peut citer les alcools inférieurs notamment l'éthanol et l'isopropanol et le propylène glycol.
Comme gélifiants hydrophiles utilisables dans l'invention, on peut citer les polymères carboxyvinyliques (carbomer® ), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Selon l'un des modes de réalisation de l'invention, on associe au moins composé de formule (I) ou ses dérivés tels que décrits dans ce qui précède, en tant qu'agent pour lutter contre les signes du vieillissement de la peau et/ou des muqueuses, à au moins un agent stimulant la synthèse de certaines macromolécules dermiques et/ou épidermiques, et/ou au moins un agent inhibant leur dégradation, et/ou au moins un agent stimulant la prolifération des fibroblastes et/ou des kératinocytes et/ou au moins un agent stimulant la différenciation des kératinocytes.
En effet, il peut être avantageux de combiner l'activité de stimulation de l'activité de protéases du stratum corneum par les composés de formule (I) selon l'invention à l'inhibition de certaines enzymes protéolytiques, notamment actives au niveau du derme et pouvant avoir une activité néfaste pour le maintien des propriétés de la peau. Il est également avantageux de renforcer l'activité sur la différenciation et la fonction barrière des agents selon l'invention par des agents modulant la différenciation et/ou la prolifération des cellules de la peau.

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés, tels que l'ascorbyl glucoside (vendu par la société Hayashibara ; les peptides de synthèse tels que la iamin, le palmitoyle de tripeptide glycine-histidine-lysine vendu sous la dénomination « Biopeptide CL » par la société SEDERMA ; les peptides extraits de végétaux, tels que l'hydrolysat de soja commercialisé par la société COLETICA sous la dénomination commerciale Phytokine® ; les extraits de fibres de soja, tel que celui vendu sous la dénomination « Raffermine » par la société SILAB ; les hormones végétales telles que les auxines et les lignanes ; le palmitoyle de pentapeptide lysine-thréonine-thréonine-lysine-sérine vendu notamment sous la dénomination « MATRIXYL » par la société SEDERMA ; le diméthyl amino éthanol ; les extraits de rizhome de Bupleurum Chinensis, tels que ceux vendus sous les dénominations « PLEURIMINCYL », « LIPOCARE » par la société SEDERMA ; les hydrolysats de protéine de blé acylés notamment par un groupement palmitoyle, tel que celui vendu sous la dénomination « LIPACID PVB » par la société SEPPIC ; la créatine ; le coenzyme Q10 ; le rétinol, le dipalmitoyl hydroxyproline, notamment commercialisé par la société SEPPIC sous la dénomination « SEPILIFT DPHP », les extraits de trèfle rouge (*trifolium pratense*) contenant des isoflavones ;
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur la synthèse de composés présents au niveau de la jonction dermo-épidermique (tels que les collagènes VII et/ou IV) et /ou la laminine, tels que le dipalmitoyl hydroxyproline, notamment commercialisé par la société SEPPIC sous la dénomination « SEPPILIFT DPHP », le sulfate de phytostérol , tel que celui commercialisé par la société VINCIENCE sous la dénomination « PHYTOCOHESINE» ;
- soit sur l'inhibition des métalloprotéinases (métalloprotéinases matricielles ou MMP) telles que plus particulièrement les MMP 1, 2, 3, 9. On peut citer: les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB® ), de trèfle rouge (commercialisé par exemple par la société SEDERMA sous la dénomination « STEROCARE® »), de lin, de kakkon ou de sauge ; les extraits de cucurma longa ; les extraits de Siegesbeckia (commercialisé par exemple par la société Sederma) ;
- soit sur l'inhibition des sérine protéases telles que l'élastase leucocytaire ou la cathepsine G. On peut citer: l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

D'autres agents inhibant les protéases pouvant être associés selon l'invention sont des inhibiteurs de l'activation du plasminogène, tel que par exemple l'acide tranexamique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G® .
Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine® ) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; un extrait peptidique de lupin tel que celui commercialisé par la société SILAB sous la dénomination commerciale Structurine® ; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; et les lignanes tels que le sécoisolaricirésinol.

La composition selon l'invention renfermant un ou plusieurs des composés ci-dessus convient particulièrement bien à une utilisation dans la prévention ou le traitement des signes cutanés du vieillissement, en particulier de la perte de fermeté et/ou d'élasticité de la peau.

L'application peut être quotidienne ou biquotidienne, et être répétée pendant plusieurs jours, plusieurs semaines et/ou plusieurs mois.

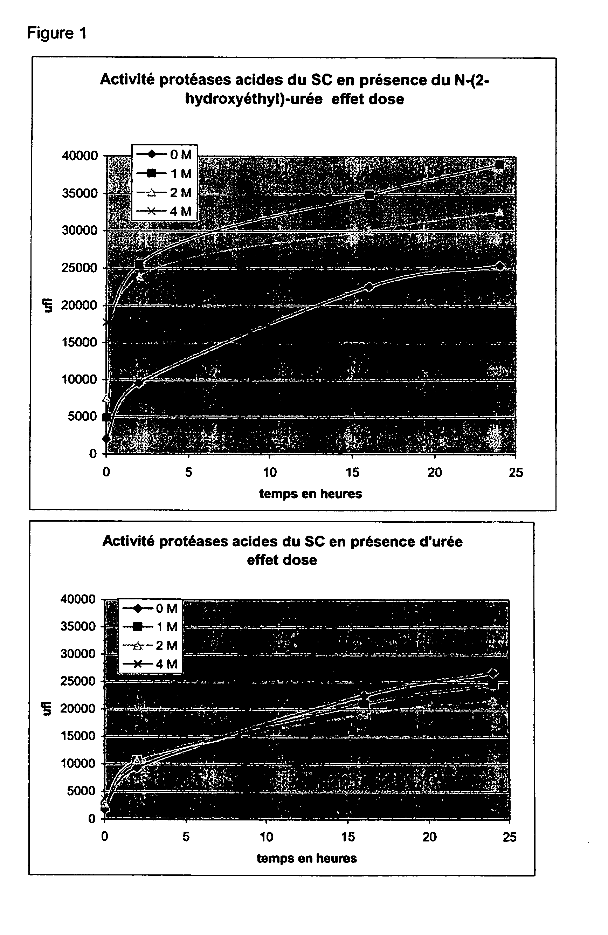

D'autres caractéristiques et avantages de l'invention apparaîtront à la lecture des exemples qui suivent.
Dans ces exemples on se référera à l'unique figure en annexe qui représente le dosage de l'activité protéasique des protéines du SC (résultats exprimés en unité de fluorescence)

### Exemple 1 : Activation de protéases acides

On évalue la propriété d'activer certaines protéases acides du SC (stratum comeum). La mesure de cette activité se fait grâce à un dosage par fluorimétrie à l'aide du kit Enzchek (Molecular Probes). Ce protocole utilise un substrat Bodipyfl-caséine qui lorsqu'il est hydrolysé libère de la fluoresoenoe. La fluorescence libérée est directement proportionnelle à l'activité protéasique. Elle est lue directement en plaque 96 puits au spectrofluorimètre à 750V à 485 nm en excitation et 535 nm en émission.
On compare les résultats obtenus avec le N-(2-hydroxyéthyl)-urée (composé selon l'invention) à ceux de l'urée.

### Mode opératoire :

Les molécules sont préparées à 0, 1, 2 et 4 M en tampon acétate 0.1 M ; pH5.0.
Un extrait enzymatique est préparé à partir de poudres acétoniques.
2 ml de tampon PBS +0.1% de TritonX100 sont mis en contact 1 h dans de la glace pilée avec 200 mg de poudres acétoniques de Stratum Comeum. Le mélange est ensuite broyé au potter puis centrifugé 10 min à 15 000g à 4°C. Le surnageant est recueilli. Le substrat Enzchek dilué au 1/200 est incorporé dans chaque solution contenant les molécules. Les essais sont répétés trois fois.
Le mélange réactionnel est réalisé directement en plaque blanche.
10µl d'extrait enzymatique sont ajoutés à 200µl de solution contenant le substrat à 0, 1, 2 ou 4M. Les lectures sont réalisées à t0, t 2h, t 16h et t 24h.

### Les résultats sont représentés sur la figure en annexe

L'urée à 1et 2M a un effet légèrement activateur sur les protéases acides jusqu'à deux heures après le début de l'incubation.
En revanche pour le N-(2-hydroxyéthyl)-urée, on note une augmentation importante de l'activité pour les concentrations de 1 et 2 M par rapport au témoin. Cette augmentation est durable dans le temps. A 4M on observe une diminution nette de l'activité quel que soit le temps étudié.

## Revendications

1. Utilisation non-thérapeutique d'au moins un composé de formule (I) suivante 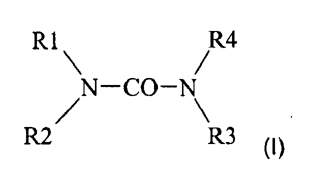 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle, ainsi que leurs sels, leurs solvats, et leurs isomères ;
comme agent pour réduire les pigmentations actiniques, le composé de formule I étant dans une composition cosmétique contenant un milieu physiologiquement acceptable.

2. Utilisation d'au moins un composé de formule (I) selon la revendication 1, **caractérisée en ce que** dans la formule (I) R₁ désigne un groupe hydroxyalkyle en C₂-C₆ et R₂, R₃, R₄ désignent, indépendamment l'un de l'autre, un atome d'hydrogène ou un groupe alkyle en C₁-C₄.

3. Utilisation selon l'une au moins des revendications 1 ou 2, **caractérisée en ce que** dans la formule (I) R1 désigne un groupe hydroxyalkyle en C₂-C₆ comprenant de 1 à 5 groupes hydroxyles, et R2, R3, R4 désignent un atome d'hydrogène.

4. Utilisation selon l'une au moins des revendications 1 à 3, **caractérisée en ce que** dans la formule (I) R1 désigne un groupe hydroxyalkyle en C2-C6 comprenant 1 groupe hydroxyle.

5. Utilisation selon l'une au moins des revendications 1 à 4, **caractérisée en ce que** R₁ désigne un groupe hydroxyalkyle en C2-C4 comprenant 1 groupe hydroxyle et R₂, R₃, R₄ désignent un atome d'hydrogène.

6. Utilisation selon l'une au moins des revendications 1 à 5, **caractérisé en ce que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée; le N-méthyl-N'-(1-hydroxy-2-méthyl-2-propyl)- urée ; le N-(1-hydroxy-2-méthyl-2-propyl)- urée ; le N-(1,3-dihydroxy-2-propyl)- urée ; le N-(tris-hydroxyméthyl-méthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée ; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)- urée ; et leurs mélanges.

7. Utilisation selon l'une au moins des revendications 1 à 6, **caractérisée en ce que** le composé de formule (I) est le N-(2-hydroxyéthyl)-urée.

8. Utilisation non-thérapeutique d'un composé de formule (I) tel que défini dans l'une au moins des revendications 1 à 7, comme agent pour stimuler l'activité d'au moins une enzyme protéolytique du stratum corneum choisie dans le groupe comprenant:
• Des protéases à sérine telles que la SCCE, la SCTE, la matriptase MT-SP1, les « proprotein convertases » ou la PEP1 (« profilaggrin endoproteinase 1 ») ;
• Des protéases à acide aspartique telles que les cathepsine D, E ou la SASPase;
• Des protéases à cystéine telle que les cathepsine B, H, L , L2 , les calpaïnes ou la caspase 14 ;
• Des métalloprotéases telle que la MMP19 ou les carboxypeptidases.

9. Utilisation selon l'une au moins des revendications 1 à 7, **caractérisée en ce que** la composition est une composition cosmétique pour application topique sur la peau et/ou les muqueuses.

10. Utilisation non-thérapeutique d'un composé de formule (I) tel que défini dans l'une au moins des revendications 1 à 7, comme agent pour réguler la différenciation épidermique dans une peau âgée.

11. Utilisation selon la revendication 10, **caractérisée en ce que** le composé de formule (I) inhibe la diminution de la quantité d'acides aminés libres dans le stratum corneum liée à l'âge.

12. Utilisation selon la revendication 11, **caractérisée en ce que** le composé de formule (I) diminue l'accumulation de protéines anormales liée au vieillissement.

## Patentansprüche

1. Nichttherapeutische Verwendung von mindestens einer Verbindung der folgenden Formel (I) 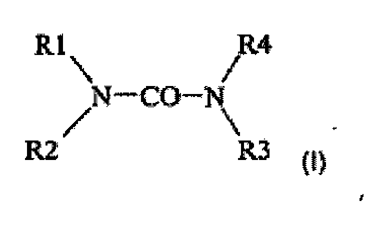 worin:
R₁, R₂, R₃ und R₄ jeweils unabhängig voneinander ein Wasserstoffatom, eine C₁-C₄-Alkylgruppe oder eine C₂-C₆-Hydroxyalkylgruppe, die 1 bis 5 Hydroxylgruppen enthalten kann, bedeuten, wobei mindestens einer der Reste R₁ bis R₄ eine Hydroxyalkylgruppe bedeutet,
sowie ihren Salzen, ihren Solvaten und ihren Isomeren; als Mittel zum Verringern von durch Strahlen hervorgerufenen Pigmentierungen, wobei die Verbindung der Formel I in einer Kosmetikzusammensetzung vorliegt, die ein physiologisch unbedenkliches Medium enthält.

2. Verwendung von mindestens einer Verbindung der Formel (I) nach Anspruch 1, **dadurch gekennzeichnet, dass** in Formel (I) R₁ eine C₂-C₆-Hydroxyalkylgruppe bedeutet und R₂, R₃, R₄ unabhängig voneinander ein Wasserstoffatom oder eine C₁-C₄-Alkylgruppe bedeuten.

3. Verwendung nach mindestens einem der Ansprüche 1 oder 2, **dadurch gekennzeichnet, dass** in Formel (I) R₁ eine C₂-C₆-Hydroxyalkylgruppe mit 1 bis 5 Hydroxylgruppen bedeutet und R₂, R₃, R₄ ein Wasserstoffatom bedeuten.

4. Verwendung nach mindestens einem der Ansprüche 1 bis 3, **dadurch gekennzeichnet, dass** in Formel (I) R₁ eine C₂-C₆-Hydroxyalkylgruppe mit 1 Hydroxylgruppe bedeutet.

5. Verwendung nach mindestens einem der Ansprüche 1 bis 4, **dadurch gekennzeichnet, dass** R₁ eine C₂-C₄-Hydroxyalkylgruppe mit 1 Hydroxygruppe bedeutet und R₂, R₃, R₄ ein Wasserstoffatom bedeuten.

6. Verwendung nach mindestens einem der Ansprüche 1 bis 5, **dadurch gekennzeichnet, dass** die Verbindung der Formel (I) aus den Folgenden ausgewählt ist: N-(2-Hydroxyethyl)harnstoff; N-(2-Hydroxypropyl)harnstoff, N-(3-Hydroxypropyl)harnstoff; N-(2,3-Dihydroxypropyl)harnstoff; N-(2,3,4,5,5-Pentahydroxyhexyl)harnstoff; N-Methyl-N-(1,3,4,5,6-pentahydroxy-2-hexyl)harnstoff; N-Methyl-N'-(1-hydroxy-2-methyl-2-propyl)harnstoff; N-(1-Hydroxy-2-methyl-2-propyl)harnstoff; N-(1,3-Dihydroxy-2-propyl)harnstoff; N-(Trishydroxymethylmethyl)harnstoff; N-Ethyl-N'-(2-hydroxyethyl)harnstoff, N,N-Bis-(2-hydroxyethyl]harnstoff; N,N'-Bis-(2-hydroxyethyl)harnstoff; N,N-Bis-(2-hydroxypropyl)harnstoff; N,N'-Bis-(2-hydroxypropyl)harnstoff; N,N-Bis-(2-hydroxyethyl)-N'-propylharnstoff; N,N-Bis-(2-hydroxy-propyl)-N'-(2-hydroxyethyl)harnstoff; N-tert.-Butyl-N'-(2-(hydroxyethyl)-N'-(2-hydroxypropyl)harnstoff; N-(1,3-Dihydroxy-2-propyl)-N'-(2-hydroxyethyl)harnstoff; N,N-Bis-(2-hydroxyethyl)-N',N'-dimethylharnstoff; N,N,N',N'-Tetrakis-(2-hydroxyethyl)harnstoff; N',N'-Bis-(2-hydroxyethyl)-N',N'-bis-(2-hydroxypropyl)harnstoff und ihren Mischungen.

7. Verwendung nach mindestens einem der Ansprüche 1 bis 6, **dadurch gekennzeichnet, dass** es sich bei der Verbindung der Formel (I) um N-(2-Hydroxyethyl)harnstoff handelt.

8. Nichttherapeutische Verwendung einer Verbindung der Formel (I) wie in mindestens einem der Ansprüche 1 bis 7 definiert als Mittel zum Stimulieren der Aktivität von mindestens einem proteolytischen Enzym des Stratum corneum, ausgewählt aus der Gruppe, die Folgendes umfasst:
• Serinproteasen wie SCCE, SCTE, Matriptase MT-SP1, die "proprotein convertases" oder PEP1 ("profilaggrin endoproteinase 1");
• Asparaginsäureproteasen wie Cathepsin D, E oder SASPase;
• Cysteinproteasen wie Cathepsin B, H, L, L2, die Calpaine oder Caspase 14;
• Metalloproteasen wie MMP19 oder die Carboxypeptidasen.

9. Verwendung nach mindestens einem der Ansprüche 1 bis 7, **dadurch gekennzeichnet, dass** es sich bei der Zusammensetzung um eine Kosmetikzusammensetzung für die topische Anwendung auf die Haut und/oder die Schleimhäute handelt.

10. Nichttherapeutische Verwendung einer Verbindung der Formel (I) wie in mindestens einem der Ansprüche 1 bis 7 definiert als Mittel zum Regulierten der Epidermisdifferenzierung in einer gealterten Haut.

11. Verwendung nach Anspruch 10, **dadurch gekennzeichnet, dass** die Verbindung der Formel (I) die altersbedingte Verringerung der Menge an freien Aminosäuren im Stratum corneum hemmt.

12. Verwendung nach Anspruch 11, **dadurch gekennzeichnet, dass** die Verbindung der Formel (I) die alterungsbedingte Anhäufung von abnormen Proteinen verringert.

## Claims

1. Nontherapeutic use of at least one compound of following formula (I): 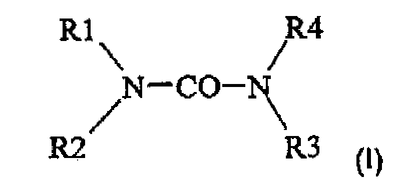 in which:
R1, R2, R3 and R4 each represent, independently of one another, a hydrogen atom, a C₁-C₄ alkyl group or a C₂-C₆ hydroxyalkyl group which can comprise from 1 to 5 hydroxyl groups, where at least one of the R1 to R4 radicals represents a hydroxyalkyl group,
and their salts, their solvates and their isomers,
as agent for reducing actinic pigmentations, the compound of formula I being in a cosmetic composition comprising a physiologically acceptable medium.

2. Use of at least one compound of formula (I) according to Claim 1, **characterized in that**, in the formula (I), R1 denotes a C₂-C₆ hydroxyalkyl group and R2, R3 and R4 denote, independently of one another, a hydrogen atom or a C₁-C₄ alkyl group.

3. Use according to at least one of Claims 1 and 2, **characterized in that**, in the formula (I), R1 denotes a C₂-C₆ hydroxyalkyl group comprising from 1 to 5 hydroxyl groups and R2, R3 and R4 denote a hydrogen atom.

4. Use according to at least one of Claims 1 to 3, **characterized in that**, in the formula (I), R1 denotes a C₂-C₆ hydroxyalkyl group comprising 1 hydroxyl group.

5. Use according to at least one of Claims 1 to 4, **characterized in that** R1 denotes a C₂-C₄ hydroxyalkyl group comprising 1 hydroxyl group and R2, R3 and R4 denote a hydrogen atom.

6. Use according to at least one of Claims 1 to 5, **characterized in that** the compound of formula (I) is chosen from N-(2-hydroxyethyl)urea; N-(2-hydroxy-propyl)urea; N-(3-hydroxypropyl)urea; N-(2,3-dihydroxypropyl)urea; N-(2,3,4,5,6-pentahydroxy-hexyl)urea; N-methyl-N-(1,3,4,5,6-pentahydroxy-2-hexyl)urea; N-methyl-N'-[1-hydroxy-2-methyl-2-propyl)urea; N-(1-hydroxy-2-methyl-2-propyl)urea; N-(1,3-dihydroxy-2-propyl)urea; N-[tris(hydroxymethyl)methyl]urea; N-ethyl-N'-(2-hydroxyethyl)urea; N,N-bis(2-hydroxyethyl)urea; N,N'-bis(2-hydroxy-ethyl)urea; N,N-bis(2-hydroxypropyl)urea; N,N'-bis(2-hydroxypropyl)urea; N,N-bis(2-hydroxyethyl)-N'-propyl-urea; N,N-bis(2-hydroxypropyl)-N'-(2-hydroxyethyl)urea; N-(tert-butyl)-N'-(2-hydroxyethyl)-N'-(2-hydroxypropyl)urea; N-(1,3-dihydroxy-2-propyl)-N'-(2-hydroxy-ethyl)urea; N,N-bis(2-hydroxyethyl)-N',N'-dimethylurea; N,N,N',N'-tetrakis(2-hydroxyethyl)urea; N',N'-bis(2-hydroxyethyl)-N',N'-bis(2-hydroxypropyl)urea; and their mixtures.

7. Use according to at least one of Claims 1 to 6, **characterized in that** the compound of formula (I) is N-(2-hydroxyethyl)urea.

8. Nontherapeutic use of a compound of formula (I) as defined in at least one of Claims 1 to 7, as agent for stimulating the activity of at least one proteolytic enzyme of the stratum corneum, chosen from the group consisting of:
• serine proteases, such as SCCE, SCTE, matriptase/MT-SP1, proprotein convertases or PEP1 (profilaggrin endoproteinase 1);
• aspartic acid proteases, such as cathepsin D, cathepsin E or SASPase;
• cysteine proteases, such as cathepsin B, cathepsin H, cathepsin L, cathepsin L2, calpaines or caspase-14;
• metalloproteases, such as MMP19 or carboxypeptidases.

9. Use according to at least one of Claims 1 to 7, **characterized in that** the composition is a cosmetic composition for topical application to the skin and/or mucous membranes.

10. Nontherapeutic use of a compound of formula (I) as defined in at least one of Claims 1 to 7, as agent for regulating epidermal differentiation in aged skin.

11. Use according to Claim 10, **characterized in that** the compound of formula (I) inhibits the reduction in the amount of free amino acids in the stratum corneum related to age.

12. Use according to Claim 11, **characterized in that** the compound of formula (I) reduces the age-related accumulation of abnormal proteins.
